Europäisches Patentamt

European Patent Office

Office européen des brevets

(11) Veröffentlichungsnummer: **0 340 609**
**A2**

## (12) EUROPÄISCHE PATENTANMELDUNG

(21) Anmeldenummer: 89107456.9

(22) Anmeldetag: 25.04.89

(51) Int. Cl.⁴: **C12P 19/34 , C07H 1/08 , C12M 1/00 , C12Q 1/68**

(30) Priorität: 06.05.88 CH 1740/88

(43) Veröffentlichungstag der Anmeldung:
08.11.89 Patentblatt 89/45

(84) Benannte Vertragsstaaten:
**AT BE CH DE FR GB IT LI NL SE**

(71) Anmelder: **F. HOFFMANN-LA ROCHE AG**
**Postfach 3255**
**CH-4002 Basel(CH)**

(72) Erfinder: **Kiefer, Hansruedi, Dr.**
**Habermarkweg 7**
**CH-4125 Riehen(CH)**

(74) Vertreter: **Buntz, Gerhard et al**
**Grenzacherstrasse 124 Postfach 3255**
**CH-4002 Basel(CH)**

(54) **Vorrichtung zur Isolation von Nukleinsäuren.**

(57) Vorrichtung zur Isolation von Nukleinsäuren aus Zell-oder Gewebeextrakten durch ein Fällungsverfahren, mit Reaktionsbehältern (31) zur Durchführung der Fällungsreaktion und nachfolgender Waschzyklen, über Filter (32) an die Reaktionsbehälter angeschlossenen Flüssigkeitsleitungen (19-21) für den Zu- und Abfluss von Lösungsmittel und Reagenzien, in den Leitungen vorhandenen Ventilen (15-18, 33-35) zur Steuerung des Zu- und Abflusses, sowie einer Einrichtung zum Bewegen der Reaktionsbehälter.

Fig.1

EP 0 340 609 A2

## Vorrichtung zur Isolation von Nukleinsäuren

Die Erfindung betrifft eine Vorrichtung zur Isolation von Nukleinsäuren aus Zell- oder Gewebeextrakten durch ein Fällungsverfahren, mit Reaktionsbehältern zur Durchführung der Fällungsreaktion und gegebenenfalls nachfolgender Waschzyklen, an die Reaktionsbehälter angeschlossenen Flüssigkeitsleitungen für den Zu- und Abflusss von Lösungsmittel, Reagenzien und dergleichen, in den Leitungen vorhandenen Ventilen zur Steuerung des Zu- und Abflusses und einer Einrichtung zum Bewegen der Reaktionsbehälter.

Bekannte Verfahren zur Isolierung von Nukleinsäuren beruhen auf einem Zweiphasensystem, dessen Phasen zuerst durchmischt und anschliessend wieder getrennt werden müssen. Zur Trennung wird üblicherweise zentrifugiert. Eine Vorrichtung zur Isolation von Nukleinsäuren ist somit nach herkömmlicher Technik eine Zentrifuge.

Ein neues Verfahren (vgl. Schweizerische Patentanmeldung Nr. 1573/87 vom 24. April 1987) besteht in der Fällung der Nukleinsäuren mit einem wasserlöslichen Keton aus einem geeignet behandelten Zell- oder Gewebeextrakt.

Der Erfindung liegt die Aufgabe zugrunde, eine Vorrichtung zur Isolierung von Nukleinsäuren bereitzustellen, mit dem die neue Fällungsmethode durchgeführt werden kann.

Erfindungsgemäss wird dies erreicht durch eine Vorrichtung der eingangs erwähnten Art, die sich dadurch auszeichnet, dass Filter vorgesehen sind, über die die Leitungen zum Abfiltrieren der präzipitierten Nukleinsäuren mit den Reaktionsbehältern verbunden sind.

Nach einer bevorzugten Ausführungsform ist eine Reihe von gleichartigen Behältern mit entsprechenden Leitungen, Ventilen und Filtern vorgesehen. Nach einer weiteren Ausführungsform der Erfindung besteht die Einrichtung zum Bewegen der Reaktionsbehälter aus einem Traggestell in dem die Reaktionsbehälter angeordnet sind und das drehbar aufgehängt ist derart, dass die Reaktionsbehälter gekippt werden können.

Im folgenden wird an Hand der beiliegenden Zeichnungen ein Ausführungsbeispiel der Erfindung beschrieben. Es zeigen

Fig. 1 eine schematische perspektivische Ansicht der Vorrichtung,

Fig. 2 ein schematisches Leitungsdiagramm der Vorrichtung nach Figur 1,

Fig. 3 eine schematische perspektivische Darstellung des Traggestells für die Reaktionsbehälter.

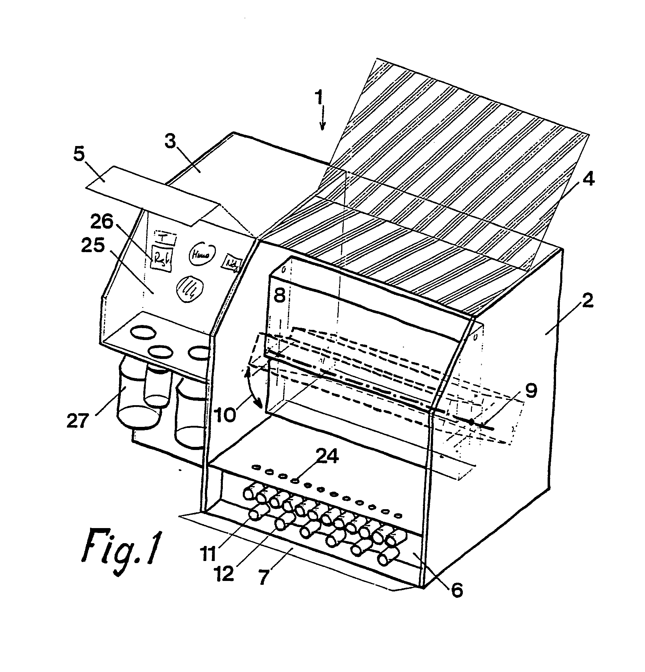

Wie in Figur 1 gezeigt besitzt die Vorrichtung ein Gehäuse 1, das aus einem grösseren Abteil 2 und einem kleineren Abteil 3 besteht. Beide Abteile besitzen auf ihrer Vorderseite jeweils einen Deckel 4, 5, durch die sie vor allem von der Vorderseite zugänglich sind. Das grössere Abteil 2 besitzt ausserdem ein unteres Fach 6, das durch eine Klappe 7 zugänglich ist.

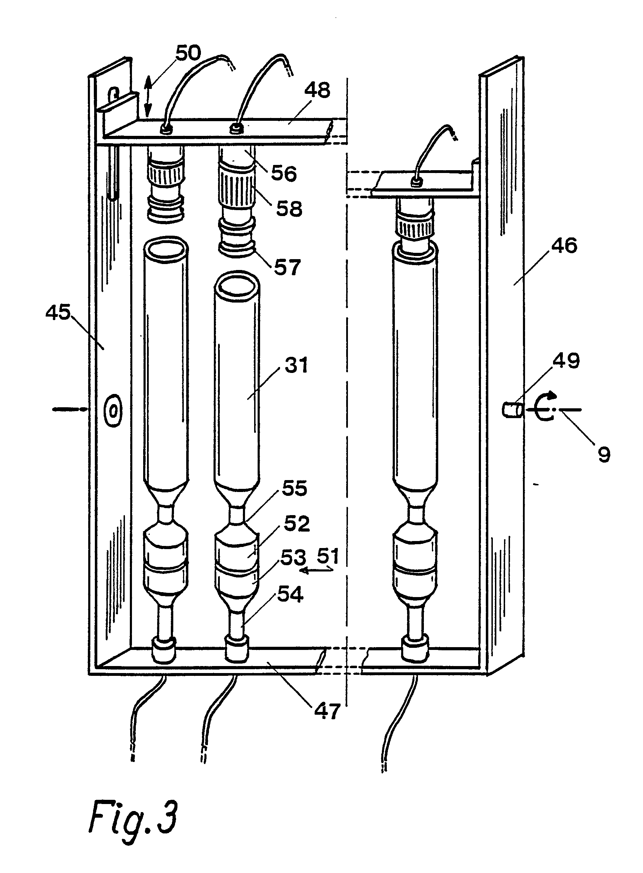

Im Inneren des Abteils 2 befindet sich ein Traggestell 8, das zur Aufnahme von Reaktionsbehältern dient und das an zwei einander gegenüberliegenden Punkten drehbar aufgehängt ist, so dass es um eine Achse 9 gekippt werden kann. Die Kippbewegung ist durch die gestrichelte Darstellung des Gestells 8 und durch den Pfeil 10 angegeben. Der Aufbau des Traggestells 8 ist in Figur 3 genauer gezeigt.

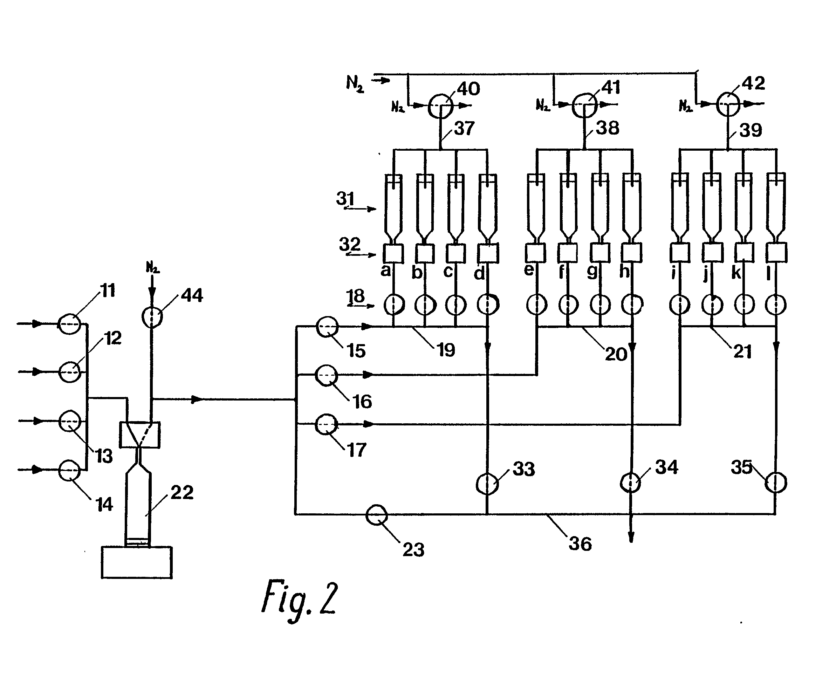

Im unteren Fach 6 befindet sich eine Anzahl von Ventilen 11, 12 etc., deren Funktion aus der folgenden Figur 2 näher ersichtlich ist. Zwischem dem unteren Fach 6 und dem oberen Teil des Abteils 2 befinden sich Durchgangsöffnungen 24, durch die die Verbindungsleitungen von den Ventilen zum Traggestell 8 führen. Diese Verbindungsleitungen sind in Figur 1 der Uebersichtlichkeit halber nicht gezeigt. Sie gehen schematisch aus Figur 2 hervor und sind in der Vorrichtung so geführt, dass sie genügend Länge und Flexibilität aufweisen, um die Kippbewegung des Traggestells 8 zu ermöglichen. Die Führung der Leitungen, die im wesentlichen aus Teflon®-Schläuchen bestehen, ist unkritisch und kann von jedem Fachmann in beliebiger Art und Weise vorgenommen werden.

Das kleinere Abteil 3 dient zur Aufnahme der Steuerungselektronik (nicht gezeigt) und weist eine Wand 25 mit Bedienungs- und Anzeigeorganen 26 auf. Auf dem Boden des Abteils 3 befinden sich Aufnahmevorrichtungen zum Einsetzen von Vorratsbehältern 27 mit den für das durchzuführende Fällungsverfahren erforderlichen Reagenzien und Waschflüssigkeiten. Von diesen Vorratsbehältern 27 führen (nicht gezeigte) Schlauchleitungen über eine im Inneren des Abteils 3 befindliche Pumpe (Motor-Bürette in Figur 2) zu den Ventilen 11, 12 etc..

Das Abteil 3 enthält eine im wesentlichen konventionelle elektronische Steuerung für die Ventile und den Antriebsmotor für das Traggestell 8, d.h. also eine Ablaufsteuerung für das gesamte Isolationsverfahren.

Aus Figur 2 ist die Zuordnung der Flüssigkeitsleitungen zueinander und ihre Verbindungen zu den anderen wesentlichen Elementen der Vorrichtung ersichtlich. Zwölf Reaktionsbehälter 31a-l zusammen mit ihnen zugeordneten Filtern 32a-l sind im Traggestell 8, das hier durch eine gestrichelte Linie angedeutet ist, angeordnet. Die Behälter sind in drei Gruppen zu je vier Behältern und vier Filtern gegliedert.

Die Kombination aus Reaktionsbehälter 31a und Filter 32a ist über eine Leitung mit einem Ventil 18a verbunden. Das gleiche gilt für die Kombination 31b/32b usw.. Die Ventilgruppe 18a-d ist über eine gemeinsame Leitung 19 über ein Einlassventil 15 mit dem Ausgang einer Motorbürette 22 verbunden. Die Leitung 19 verbindet die Ventile 18a-d ausserdem über ein Auslassventil 33 mit einer Entsorgungsleitung 36. In gleicher Weise ist die Ventilgruppe 18e-h über eine gemeinsame Leitung 20 einerseits über ein Einlassventil 16 mit dem Ausgang der Motorbürette 22 und über ein Auslassventil 34 mit der Entsorgungsleitung 36 verbunden. Die Ventilgruppe 18i-l ist über eine gemeinsame Leitung 21 und über ein Einlassventil 17 mit der Motorbürette 22 und über ein Auslassventil 35 mit der Entsorgungsleitung 36 verbunden. Durch diese Art der Ventilkombination können die Behälter 31 sowohl gruppenweise als auch einzeln mit Flüssigkeit ver- und entsorgt werden.

Die Motorbürette 22 ist eingangsseitig über Einlassventile 11, 12, 13 und 14 mit den Vorratsbehältern für Reagenzien und Waschflüssigkeiten verbunden. Die Verbindungsleitung zwischen der Motorbürette 22 und den Einlassventilen 15-17 ist ausserdem über ein Ventil 23 mit der Entsorgungsleitung 36 verbunden. Dieses Ventil 23 ist im Wechsel geschlossen, solange eines der Ventile 15-17 geöffnet ist. Das Ventil 23 ist dagegen geöffnet wenn alle drei Ventile 15-17 geschlossen sind. Dies hat zum Zweck, dass die Motorbürette nicht gegen geschlossene Ventile arbeiten muss. Das Ventil 23 hat also die Funktion eines Sicherheitsventils.

Zu den Reaktionsbehältern 31 führen ausserdem von oben noch Leitungen 37-39, die über Ventile 40-42 mit einer Druckleitung 43 für Stickstoff verbunden sind. Wenn das Ventil 40 geöffnet ist, wird Stickstoff unter Druck in die Reaktionsgefässe 31a-d geleitet und dadurch der Inhalt der Reaktionsgefässe durch die zugehörigen Filter ausgepresst. Das gleiche gilt für die anderen Gruppen von Reaktionsbehältern beim Oeffnen der Ventile 41 oder 42. Der ausgepresste Inhalt gelangt über die Ventile 18 zu den Leitungen 19, 20, 21 und von dort über die Ventile 33, 34, 35 zur Entsorgungsleitung 36.

Der Ausgang der Bürette 22 ist über ein Ventil 44 mit einer Druckleitung für Stickstoff verbunden. Das Ventil 44 ist immer dann geöffnet wenn die Bürette an ihrem Ausgang keinen Druck erzeugt. Auf diese Weise steht die Zuleitung zu den Ventilen 15-17 ständig unter Stickstoffatmosphäre. Wenn der Kolben der Bürette 22 vorgeschoben wird, ist das Ventil 44 geschlossen. Der Ablauf der Flüssigkeitsbewegungen von den Vorratsbehältern über die Ventilkombinationen zu den Reaktionsbehältern und von diesen weg zur Entsorgungsleitung ist am besten aus Figur 2 ersichtlich. Die Funktion ist wie folgt:

Die einzelnen Reaktionsbehälter 31 werden in das Gestell 8 so eingesetzt, dass sie dicht an den entsprechenden Filter angeschlossen sind. Anschliessend werden sie mit Zell- oder Gewebeextrakten und ggf. einem ersten Reagens gefüllt und auch mit ihren oberen Oeffnungen dicht mit den Leitungen verbunden, die zur Stickstoff-Druckleitung führen. Wenn alle Reaktionsgefässe gefüllt sind, wird das Ablaufprogramm eingeschaltet. Zu Beginn des Programms befindet sich der Kolben der Motorbürette 22 in seiner vorgeschobenen Stellung, um Flüssigkeit ansaugen zu können. Auf einen Befehl von der Steuerung öffnet das Ventil 11, das die von einem Vorratsbehälter kommende Leitung kontrolliert, der ein Keton/Wasser-Gemisch enthält. Gleichzeitig beginnt die Bürette 22 den Ansaugvorgang und saugt eine Menge von 50 ml des Keton/Wasser-Gemisches an.

Nachdem der Ansaugvorgang beendet ist, schaltet der Hahn um, und das Ventil 15 wird geöffnet. Gleichzeitig ist das Ventil 18a geöffnet, während das Ventil 33 geschlossen ist. Die Bürette erhält nun den Befehl zum Ausstossen der angesaugten Flüssigkeit die über die Ventile 15 und 18a, sowie über den Filter 32a in das Reaktionsgefäss 31a gelangt. Danach wird das Ventil 18a geschlossen und der gleiche Vorgang mit dem Ventil 18b wiederholt. In gleicher Weise geht es mit den anderen Ventilen, bis die Reaktionsbehälter 31a-d gefüllt sind. Entsprechend verfährt man mit den Reaktionsbehältern 31e-h mit den Ventilen 16, sowie mit sequentieller Oeffnung der Ventile 18e-h.

Wenn alle Reaktionsbehälter gefüllt sind, wird das Gemisch durch regelmässige Kippbewegungen des Traggestells 8 bei Raumtemperatur gut durchmischt. Nach einer für die Fällung der Nukleinsäuren ausreichenden Zeit wird die Bewegung des Gestells 8 beendet, und die Ventile 33, 34, 35 werden geöffnet. Gleichzeitig werden die Ventile 18, sowie die den Stickstoff einleitenden Ventile 40-42 geöffnet. Durch den Stickstoff wird die gesamte Flüssigkeit durch die Filter 32 ausgepresst und gelangt zur Entsorgungsleitung 36.

Die ausgefällten Nukleinsäuren bleiben an den Filtern 32 zurück. Nach dem Auspressen werden die Ventile 18, 33-35 und 40-42 wieder geschlossen und die Ventile 12, 15 und 18a geöffnet. Mit der Bürette wird nun über das Ventil 12 ein Alkohol/Wassergemisch angesaugt und über die Ventile 15 und 18a in den Reaktionsbehälter 31a gepresst. In gleicher Weise wird mit den anderen Reaktionsbehältern verfahren. Auf diese Weise wird die ausgefällte Nukleinsäure gewaschen. Der Waschvorgang kann mehrfach wiederholt werden und auf Wunsch auch mit anderen Waschflussigkeiten vorgenommen werden. Auf diese Weise erhält man im Filter reine Nukleinsäure, die in Wasser gelöst werden kann.

Das Gestell 8 ist in Figur 3 im Detail gezeigt. Es besteht aus einem Rahmen aus Seitenteilen 45, 46, einer unteren Querverbindung 47 und einer oberen Querverbindung 48. An den beiden Seitenteilen sind Bolzen 49 angebracht, die die Achse 9 definieren, um die das Traggestell wie bereits erwähnt, gekippt wird.

Die obere Querverbindung ist, wie durch einen Pfeil 50 angedeutet, in der Höhe verstellbar. Zur Verstellung dient ein Hebelmechanismus, der hier nicht speziell gezeigt ist. Der Zweck der Höhenverstellung wird nachstehend näher beschrieben.

Auf der unteren Querverbindung 47 sind in gleichen Abständen voneinander 12 gleichartige Behälter 51 angeordnet, die aus je zwei miteinander verschraubten Hälften 52, 53 bestehen und über einen rohrformigen Ansatz 54 mit der Querverbindung 47 verbunden sind. Die Behälter 51 dienen zur Aufnahme der Filter 32. Auf ihrer Oberseite weisen sie eine Oeffnung 55 auf, die zur Aufnahme der Spitze der Reaktionsbehälter 31 geeignet ist. Auf der Unterseite der Querverbindung sind die Rohrleitungen 54 mit Schläuchen verbunden, die zu den Ventilen 18 führen.

Die Reaktionsbehälter 31 bestehen im vorliegenden Fall aus handelsüblichen Einwegspritzen mit entsprechendem Volumen. Wie in der Figur 3 gezeigt sind Spritzen mit verschiedener Grösse einsetzbar. ·

An der oberen Querverbindung befinden sich in konzentrischer Anordnung zu den Behältern 51 und den Reaktionsgefässen 31 12 zylindrische Halter 56, auf die Verschlussstopfen 57 zum Verschliessen der Reaktionsbehälter 31 mittels Ueberwurfschrauben 58 aufgeschraubt sind. Wie aus den beiden, auf der linken Seite der Figur 3 gezeigten Variationen ersichtlich ist, befinden sich die Verschlussstopfen 57 auf verschiedene Höhe für das Verschliessen verschieden grosser Reaktionsbehälter 31.

Die Höhenverstellbarkeit der Querverbindung 48 dient dem Oeffnen bzw. Schliessen der Behälter. Auf der linken Seite der Figur 3 ist der geöffnete Zustand gezeigt, der dadurch erreicht wird, dass die Querverbindung 48 in ihrer angehobenen Position ist. Auf der rechten Seite der Figur ist der geschlossene Zustand gezeigt, bei dem die Querverbindung 48 sich in ihrer tiefsten Lage befindet.

Zu den einzelnen Haltern 56 mit Stopfen 57 führen Verbindungsschläuche 60, die zu den Ventilen 40, 41, 42 führen. Wie bereits erwähnt, wird über diese Ventile Stickstoff zum Auspressen des Inhalts der Reaktionsbehälter zugeführt. Im Inneren der Halter münden die Verbindungsschläuche 60 in geeignete Rohrstücke (nicht gezeigt), die durch die Stopfen 57 führen.

## Ansprüche

1. Vorrichtung zur Isolation von Nukleinsäuren aus Zell- oder Gewebeextrakten durch ein Fällungsverfahren, mit Reaktionsbehältern zur Durchführung der Fällungsreaktion und gegebenenfalls nachfolgender Waschzyklen, an die Reaktionsbehälter angeschlossenen Flüssigkeitsleitungen für den Zu-und Abfluss von Lösungsmittel, Reagenzien und dergleichen, in den Leitungen vorhandenen Ventilen zur Steuerung des Zu-und Abflusses und einer Einrichtung zum Bewegen der Reaktionsbehälter, dadurch gekennzeichnet, dass Filter (32 a-l) vorgesehen sind über die die Leitungen (19, 20, 21) zum Abfiltrieren der präzipitierten Nukleinsäuren mit den Reaktionsbehältern (31-a-l) verbunden sind.

2. Vorrichtung nach Anspruch 1, dadurch gekennzeichnet, dass eine Reihe von gleichartigen Behältern mit entsprechenden Leitungen, Ventilen und Filtern vorgesehen sind.

3. Vorrichtung nach Anspruch 1, dadurch gekennzeichnet, dass die Einrichtung zum Bewegen der Reaktionsbehälter aus einem Traggestell (8) besteht, in dem die Reaktionsbehälter angeordnet sind und das drehbar aufgehängt ist derart, dass die Reaktionsbehälter gekippt werden können.

*Fig.1*

Fig. 2

Fig.3